(19) Europäisches Patentamt
European Patent Office
Office européen des brevets

(11) **EP 3 530 276 A1**

(12) **EUROPEAN PATENT APPLICATION**
published in accordance with Art. 153(4) EPC

(43) Date of publication:
**28.08.2019 Bulletin 2019/35**

(51) Int Cl.:
*A61K 35/28* *(2015.01)* *A61K 9/10* *(2006.01)*
*A61K 9/12* *(2006.01)* *A61K 38/00* *(2006.01)*
*A61K 38/48* *(2006.01)* *A61P 9/00* *(2006.01)*
*A61P 27/02* *(2006.01)* *A61P 43/00* *(2006.01)*

(21) Application number: 17861576.1

(22) Date of filing: **13.10.2017**

(86) International application number:
**PCT/JP2017/037263**

(87) International publication number:
**WO 2018/074381 (26.04.2018 Gazette 2018/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **18.10.2016 JP 2016204590**

(71) Applicants:
• **Osaka University**
**Suita-shi, Osaka 565-0871 (JP)**
• **Rohto Pharmaceutical Co., Ltd.**
**Osaka-shi**
**Osaka 544-8666 (JP)**

(72) Inventors:
• **SAWA Yoshiki**
**Suita-shi**
**Osaka 565-0871 (JP)**
• **MIYAGAWA Shigeru**
**Suita-shi**
**Osaka 565-0871 (JP)**
• **KAJITA Daisuke**
**Suita-shi**
**Osaka 565-0871 (JP)**
• **TAMADA Kotoe**
**Osaka-shi**
**Osaka 544-8666 (JP)**

(74) Representative: **Mewburn Ellis LLP**
**City Tower**
**40 Basinghall Street**
**London EC2V 5DE (GB)**

(54) **KIT FOR PREPARING DISEASE-TREATING AGENT, DISEASE-TREATING AGENT AND METHOD FOR PREPARING DISEASE-TREATING AGENT**

(57) The present invention addresses the problem of providing a disease-treating agent which exerts an excellent effect in treating diseases requiring an emergency surgery such as heart failure and which is efficacious at a certain level for a large number of patients. The present invention pertains to a kit for preparing a disease-treating agent, said kit comprising a) a fibrinogen solution, b) a thrombin solution and c) mesenchymal stem cells, in separate forms respectively. It is preferred that c) the mesenchymal stem cells are allogeneic to a subject to be treated. Also, the present invention pertains to a kit for preparing a disease-treating agent, said kit being to be used by, when in use, suspending c) the mesenchymal stem cells in either a) the fibrinogen solution or b) the thrombin solution, and then spraying the cell suspension thus obtained directly to a disease site substantially simultaneously with either b) the thrombin solution or a) the fibrinogen solution that is not used in the suspending step.

**Description**

TECHNICAL FIELD

The present invention relates to a kit for preparing an agent for treating diseases, an agent for treating diseases, and a method for preparing an agent for treating diseases.

BACKGROUND ART

Heart transplantation has been considered as the most effective procedure to treat severe heart failure caused by ischemic cardiomyopathy; however, the donor shortage is a grave problem. Use of an artificial heart is also one of the options, but an issue of complications such as infection or cerebral thorombosis has been pointed out. On the other hand, myocardial regeneration therapy using an autologous skeletal myoblast sheet has been drawing attentions as a recent new therapy (see Patent Document 1). The myocardial regeneration therapy by a skeletal myoblast sheet intends to achieve the recovery of cardiac function by creating a sheet of skeletal myoblasts collected from patient's own skeletal muscle and attaching to the surface of the heart suffering from heart failure. The myocardial regeneration therapy by an autologous skeletal muscle-derived cell sheet is free from a rejection because patient's own cells are used and is said to hardly cause complications such as severe ventricular arrhythmia. However, the therapy causes inconveniences in that the preparation requires costs and time, making the therapy unable to deal with an emergency surgery and that properties of created cell sheets are different in every patient because self-tissue-derived cells are used and effects to be obtained are also hardly consistent.

Fibrin gel using a bio-derived component has been used as, for example, a hemostat, a biotissue adhesive, and a tissue damage coating during transplantation (see Patent Documents 2 and 3). Additionally, a biotissue repairing agent containing an active ingredient in a fibrin gel (see Patent Document 4) is also known. The fibrin gel forms a coating film on the surface of an affected area when fibrinogen and thrombin, which are precursors, are sprayed on the surface of the affected area and is expected to provide effects for suppressing the leakage of an infiltrate or bleeding from the affected area and healing damaged tissues by the active ingredient; however, there has been no example where the fibrin gel is used to treat severe heart failure caused by ischemic cardiomyopathy.

PRIOR ART DOCUMENTS

Patent Document

Patent Document 1: JP 2003-306434 A

Patent Document 2: JP S58-185162 A

Patent Document 3: JP S57-153645 A

Patent Document 4: JP 2004-161649 A

SUMMARY OF INVENTION

Technical Problem

The present invention was accomplished based on the above-mentioned circumstances and an object thereof is to provide an agent for treating diseases having good effects in treating diseases requiring an emergency surgery such as heart failure and achieving a consistent effect on many patients.

Solution to Problem

The present inventors conducted extensive studies to solve the above problems and have found that, at the time of a surgery of a disease such as heart failure, when mesenchymal stem cells are suspended in a fibrinogen solution or a thrombin solution and then the obtained cell suspension is directly sprayed on a disease site substantially at the same time with either the fibrinogen solution or the thrombin solution that is not used in the suspending step, cardiac function and the like can be improved significantly. The solution directly sprayed on a disease site gels and coats the disease site, whereby the mesenchymal stem cells in the gel sufficiently act on the disease site and exert good treatment effects. The present invention was accomplished based on these findings.

The present invention accomplished to solve the above problems includes:

[1] A kit for preparing an agent for treating diseases comprising a) a fibrinogen solution, b) a thrombin solution, and c) mesenchymal stem cells in separate forms.

[2] The kit for preparing an agent for treating diseases according to [1], wherein c) the mesenchymal stem cells are allogeneic to a subject.

[3] The kit for preparing an agent for treating diseases according to [1] or [2], wherein, when in use, c) the mesenchymal stem cells are suspended in either a) the fibrinogen solution or b) the thrombin solution and then the obtained cell suspension is directly sprayed on a disease site substantially at the same time with either a) the fibrinogen solution or b) the thrombin solution that is not used in the suspending step.

[4] The kit for preparing an agent for treating diseases according to [3], wherein the directly sprayed solution gels on the disease site to be an agent for treating diseases.

[5] The kit for preparing an agent for treating diseases according to any one of [1] to [4], wherein c) the mesenchymal stem cells are frozen cells.

[6] The kit for preparing an agent for treating diseases according to any one of [1] to [5], wherein the disease is a visceral disease or an ocular disease.

[7] The kit for preparing an agent for treating diseases according to any one of [1] to [6], wherein c) the mesenchymal stem cells are prepared in such a way as to be contained in $1 \times 10^6$ to $1 \times 10^9$ cells/mL in the gel.

[8] A gel agent for treating diseases prepared by suspending c) a mesenchymal stem cell in either a) a fibrinogen solution or b) a thrombin solution and directly spraying the obtained cell suspension on a disease site substantially at the same time with either a) the fibrinogen solution or b) the thrombin solution that is not used in the suspending step.

[9] A method for preparing a gel agent for treating diseases, wherein c) mesenchymal stem cells are suspended in a) a fibrinogen solution or b) a thrombin solution and then the obtained cell suspension is directly sprayed on a disease site substantially at the same time with either a) the fibrinogen solution or b) the thrombin solution that is not used in the suspending step.

According to another aspect of the present invention, provided is a method for treating diseases such as heart diseases, wherein c) the mesenchymal stem cells are suspended in either a) the fibrinogen solution or b) the thrombin solution and then the obtained cell suspension is directly sprayed on a disease site substantially at the same time with either a) the fibrinogen solution or b) the thrombin solution that is not used in the suspending step.

Advantageous Effects of Invention

According to the kit for preparing an agent for treating diseases of the present invention, at the time of a surgery of a disease such as heart failure, when mesenchymal stem cells are suspended in either a) a fibrinogen solution or b) a thrombin solution and then the obtained cell suspension is directly sprayed on a disease site substantially at the same time with either a) the fibrinogen solution or b) the thrombin solution that is not used in the suspending step, function, structure and the like at the disease site such as heart can be notably improved. The mesenchymal stem cells hardly cause a rejection even to an allogeneic subject and thus donor's cells prepared in advance, which have been expanded and cryopreserved, can be used as the mesenchymal stem cell for the kit for preparing an agent for treating diseases of the present invention. For this reason, when compared with the case where autologous mesenchymal stem cells are prepared and used, commercialization is easier, and stable and consistent effects can be easily achieved, hence making the present invention advantageous.

BRIEF DESCRIPTION OF DRAWINGS

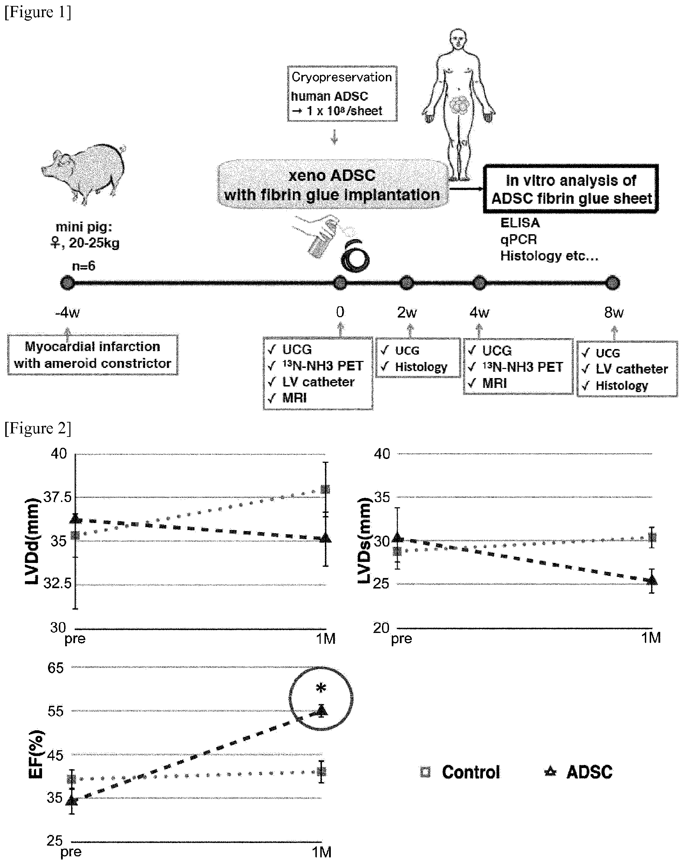

Fig. 1 shows an experimental protocol to study treatment effects of the agent for treating diseases of the present invention on mini pig myocardial infarction models.

Fig. 2 shows cardiac function evaluation by echocardiography on mini pig myocardial infarction models treated with the agent for treating diseases of the present invention.

Fig. 3 shows a protocol of evaluation on myocardial blood flow by $^{13}$N-NH$_3$-PET on mini pig myocardial infarction models treated with the agent for treating diseases of the present invention.

Fig. 4 shows coronary flow reserve of mini pig myocardial infarction models treated with the agent for treating diseases of the present invention.

Fig. 5 shows myocardial contractility (ESPVR) and diastolic function (EDPVR) of mini pig myocardial infarction models treated with the agent for treating diseases of the present invention.

Fig. 6 shows MRI results of mini pig myocardial infarction models pre- and post-treatment with the agent for treating diseases of the present invention.

Fig. 7 shows HGF secretion levels from cells in a gel.

Fig. 8 shows VEGF secretion levels from cells in a gel.

Fig. 9 shows SDF-1 secretion levels from cells in a gel.

Fig. 10 shows mRNA expression levels in cells in a gel.

Fig. 11 shows HGF and VEGF secretion levels from cells in a gel in which KN1 infusion is used.

Fig. 12 shows HGF and VEGF secretion levels from cells in a gel in which lactated Ringer's solution is used.

Fig. 13 shows mRNA expression levels in cells in a gel and in cells of adherent culture.

DESCRIPTION OF EMBODIMENTS

Hereinafter, the kit for preparing an agent for treating diseases, the agent for treating diseases and the method for preparing an agent for treating diseases of the present invention and the like are described in detail.

<Kit for preparing an agent for treating diseases>

The kit for preparing an agent for treating diseases of the present invention comprises a) a fibrinogen solution, b) a thrombin solution, and c) mesenchymal stem cells in separate forms. The kit is used by, when in use, suspending c) the mesenchymal stem cells in either a) the fibrinogen solution or b) the thrombin solution and then directly spraying the obtained cell suspension on a disease site substantially at the same time with either a) the fibrinogen solution or b) the thrombin solution that is not used in the suspending step. Hydrolysis of fibrinogen is caused by thrombin on the disease site and fibrin is produced to form a fibrin net, whereby the sprayed solution becomes gel. The thus gelled agent for treating diseases coats the disease site and provides good treatment effects on various diseases by the action of the mesenchymal stem cells contained therein. Hereinafter, each essential components of the present invention is specifically described.

[Fibrinogen]

Fibrinogen in the present invention is one of the common coagulation factors and is a glycoprotein which is converted to fibrin to produce coagulation thrombus when hydrolyzed by thrombin at the final stage of blood clotting. Fibrinogen plays a key role in the hemostatic mechanism. For example, in human, fibrinogen refers to coagulation factor I.

An acquisition method of fibrinogen in the present invention is not particularly limited but, for example, the following method is included. Plasma is separated from blood or bone marrow according to a routine method and ethanol is added to a final concentration is 8% at pH near neutrality and a low temperature around 0°C to thereby produce a precipitate. The precipitate produced is collected and dissolved in 0.05 M phosphate buffer or the like at pH 6.4. And then fibrinogen is obtained by precipitation again with 0.25 M ammonium sulfate or the like. Additionally, for obtaining a fibrinogen solution having higher coagulation performance, other coagulation factors further need to be added, and thus a method for obtaining a white precipitate (cryoprecipitate) appeared when plasma is quick-frozen and slowly melted at a low temperature of about 5°C is also included as a preferable example from the viewpoint of costs and operability. Further, a recombinant fibrinogen may also be used as the fibrinogen in the present invention.

[Thrombin]

Thrombin in the present invention refers to an endoprotease associated with blood clotting in which fibrinogen is converted to fibrin and, for example, in human, refers to a substance called activated factor II.

An acquisition method of thrombin in the present invention is not particularly limited but, for example, the following method may be included. Prothrombin is obtained by a method in which plasma is separated from blood or bone marrow according to a routine method, and a salt such as barium sulfate, calcium phosphate, magnesium hydroxide, or aluminum hydroxide is acted thereon, followed by extracting prothrombin with saline or citric acid, or by a method releasing prothrombin by the action of high pressure carbon dioxide. Activated factor X and/or calcium ion, phospholipid, and activated factor V are reacted on the obtained prothrombin to obtain thrombin. Further, a recombinant thrombin may also be used as the thrombin in the present invention.

[Fibrinogen solution and thrombin solution]

The fibrinogen solution in the present invention is a solution containing at least fibrinogen and may contain an infusion, medium, a buffer or other components within a range in which the effects of the present invention are not affected. Additionally, the thrombin solution in the present invention refers to a solution in which at least thrombin is dissolved in a suitable solvent, preferably in a solvent containing calcium ion and may contain an infusion, medium, a buffer or other components within a range in which the effects of the present invention are not affected. Further, examples of the method for preparing the thrombin solution in an easier manner include a method of obtaining a thrombin solution containing calcium ion by a method in which calcium ion and ethanol are acted on plasma in the presence of a negatively charged surface such as a glass or a ceramic and fibrin clots precipitated are removed.

For preparing the agent for treating diseases using the kit for preparing an agent for treating diseases of the present invention and sufficiently exerting treatment effects by stably immobilizing the agent on the surface of a disease site, the fibrinogen solution and the thrombin solution need to be in contact within a predetermined concentration range. When each concentration is too low, the immobilization (gelation) at the surface of a disease site requires a long period of time, hence not preferable. For quick gelation after spraying, in a case of, for example, spraying the fibrinogen solution and the thrombin solution in equal amounts, a concentration of fibrinogen is 0.1 mg/mL to 10 g/mL, preferably 0.5 mg/mL to 2.0 g/mL, more preferably 1 mg/mL to 500 mg/mL, further preferably 2 mg/mL to 200 mg/mL, and particularly preferably 5 mg/mL to 100 mg/mL. A concentration of the thrombin solution (unit/mL) is 0.1 unit/mL to 10,000 unit/mL, preferably 1 unit/mL to 2,000 unit/mL, more preferably 5 unit/mL to 1,000 unit/mL, and further preferably 10 unit/mL to 500 unit/mL. Unit for a specific gravity of the above thrombin is the unit stipulated in the Japanese Pharmacopoeia.

[Mesenchymal stem cells]

Mesenchymal stem cells in the present invention means cells having differentiation potency into cells belonging to the mesenchymal (bone cells, cardiac muscle cells, chondrocytes, tendon cells, adipocytes, etc.) and capable of proliferating while maintaining such a potency. The term mesenchymal stem cells used in the present invention means cells which are the same as stromal cells and do not particularly distinguish both from each other. Examples of the tissue containing mesenchymal stem cells include adipose tissue, umbilical cord, bone marrow, umbilical-cord blood, endometrial, placenta, dermis, skeletal muscle, periosteum, dental sac, periodontal ligament, pulp, and tooth-germ. Thus, an adipose-derived mesenchymal stem cells mean, for example, mesenchymal stem cells contained in an adipose tissue and may be termed as adipose-derived stromal cells. Of these, from the viewpoints of effects to ameliorate visceral diseases and the like by the agent for treating diseases of the present invention and availability, adipose-derived mesenchymal stem cells, umbilical cord-derived mesenchymal stem cells, bone marrow-derived mesenchymal stem cells, placenta-derived mesenchymal stem cells, and pulp-derived mesenchymal stem cells are preferable, and adipose-derived mesenchymal stem cells and umbilical cord-derived mesenchymal stem cells are more preferable.

Mesenchymal stem cells in the present invention are preferably autologous or allogeneic to a subject. The mesenchymal stem cells hardly cause a rejection even to an allogeneic subject and thus donor's cells prepared in advance are expanded, cryopreserved and can be used as the mesenchymal stem cells for the kit for preparing an agent for treating diseases of the present invention. For this reason, when compared with the case where autologous mesenchymal stem cells are prepared and used, the mesenchymal stem cells in the present invention are more preferably allogenic from the viewpoint that commercialization is easier and stable and consistent effects can be easily achieved.

Mesenchymal stem cells in the present invention also mean any cell populations containing mesenchymal stem cells. At least 20% or more, preferably 30%, 40%, 50%, 60%, 70%, 75%, 80%, 85%, 90%, 93%, 96%, 97%, 98% or 99% of such a cell population consists of mesenchymal stem cells.

Adipose tissue in the present invention means a tissue containing stromal cells containing adipocytes and microvascular cells and, for example, tissues obtained by surgical resection or aspiration from subcutaneous fat of a mammal. Adipose tissue can be obtained from subcutaneous fat. Subcutaneous fat is preferably obtained from a species same as a subject to administration of adipose-derived mesenchymal stem cells to be described later, and when considering administration to human, human subcutaneous fat is more preferable. A subcutaneous fat-supplying individual may be alive or dead, but adipose tissue used in the present invention is preferably tissues collected from a live individual. In the case of collecting from an individual, examples of the liposuction include PAL (Power-Assisted) Liposuction, elcornia laser liposuction, and body jet liposuction, and from a viewpoint of preserving the condition of cells, ultrasonic wave is preferably not used.

Umbilical cord in the present invention is a white conduit tissue connecting a fetus and the placenta, composed of umbilical vein, umbilical artery, mucous connective tissue (Wharton's Jelly), and umbilical substrate itself and containing a large number of mesenchymal stem cells. Umbilical cord is preferably obtained from a species same as a subject (subject to administration) to whom the agent for treating diseases of the present invention is used, and when considering administration of the agent for treating diseases of the present invention to human, human umbilical cord is more preferable.

Bone marrow in the present invention refers to a parenchyma filling the bone lumen and is a hematopoietic organ. Bone marrow fluid is present in the bone marrow, and the cells present therein are called bone marrow cells. Bone marrow cells contain, in addition to red-blood cells, granulocytes, megakaryocytes, lymphocytes, and adipocytes, mesenchymal stem cells, hematopoietic stem cells, vascular endothelial precursor cells and the like. Bone marrow cells can be collected from, for example, human iliac, long bone, or other bones.

Various tissues-derived mesenchymal stem cells such as adipose-derived mesenchymal stem cells, umbilical cord-derived mesenchymal stem cells, and bone marrow-derived mesenchymal stem cells in the present invention mean any cell populations containing each tissue-derived mesenchymal stem cells such as adipose-derived mesenchymal stem cells, umbilical cord-derived mesenchymal stem cells, and bone marrow-derived mesenchymal stem cells, respectively. At least 20% or more, preferably 30%, 40%, 50%, 60%, 70%, 75%, 80%, 85%, 90%, 93%, 96%, 97%, 98% or 99% of such a cell population consists of tissue-derived mesenchymal stem cells such as adipose-derived mesenchymal stem cells, umbilical cord-derived mesenchymal stem cells, and bone marrow-derived mesenchymal stem cells, respectively.

(Method for preparing mesenchymal stem cells)

The mesenchymal stem cells can be prepared by a method well known by a person skilled in the art. A method for preparing adipose-derived mesenchymal stem cells is described below as an example. Adipose-derived mesenchymal stem cells may be obtained by the production method described in, for example, U.S. Pat No. 6,777,231, and can be produced by a method including, for example, the following steps (i) to (iii):

(i) a step of obtaining a cell suspension by enzymatic digestion of adipose tissue;
(ii) a step of sedimenting cells and resuspending in suitable medium; and
(iii) a step of culturing cells on a solid surface and removing cells not binding to the solid surface.

For the adipose tissue used in the step (i), it is preferable to use those which have been washed. Washing can be carried out using a physiologically compatible saline solution (for example, phosphate buffer solution (PBS)) by vigorously stirring to cause sedimentation. This is to remove impurities (also called debris including, for example, damaged tissue, blood, and red-blood cells) contained in adipose tissue therefrom. For this reason, washing and sedimentation are repeated in general until all debris is removed from the supernatant. Remaining cells are present in the form of masses of different sizes, and for this reason it is preferable for washed cell masses to have cell junctions weaken or enzymatically treated for destruction (for example, collagenase, dispase, or trypsin) to dissociate while minimizing damages of the cells themselves. An amount of and a treatment period with an enzyme vary depending on conditions employed but are already known in the technical field concerned. In place of or in combination with such an enzymatic treatment, cell masses can be decomposed by other treatment methods such as mechanical stirring, ultrasonic energy, or thermal energy but it is preferable to carry out only by enzymatic treatment for minimizing cell damages. When an enzyme is used, it is desirable to deactivate the enzyme using medium or the like after taking a suitable period for minimizing a harmful action on cells.

The cell suspension obtained in the step (i) contains a slurry or a suspension of aggregated cells and various contaminant cells such as red-blood cells, smooth-muscle cells, endothelial cells, and fibroblast. Accordingly, the aggregated cells and these contaminant cells may subsequently be separated and removed but the removal can be achieved by adhering and washing in the step (iii) to be described later whereby such a separation and removal may be omitted. The separation and removal of contaminant cells, if performed, can be achieved by centrifugation in which cells are forcibly separated into the supernatant and the precipitation. The obtained precipitate containing contaminant cells is suspended in a physiologically compatible solvent. Suspended cells are likely to contain red-blood cells but a step of dissolution is not always necessary because red-blood cells are excluded by the selection of adhering to a solid surface to be described later. As a method for selectively dissolving red-blood cells, a method well known in the technical field concerned can be employed such as incubation in hypertonic medium or hypotonic medium by the dissolution in ammonium chloride. After dissolution, the lysate may be separated from desirable cells by, for example, filtration, centrifugal sedimentation, or density fraction.

In the step (ii), the suspended cells may be washed once or several times consecutively for enhancing the purity of mesenchymal stem cells, centrifuged, and resuspended in medium. In addition to this, the cells may be separated based on cell surface markers profile or on the size and granular properties.

Medium used for resuspension is not particularly limited as long as it can culture mesenchymal stem cells, and such a medium may be created by adding, to basal medium, serum and/or one or more serum substitutes such as albumin, transferrin, fatty acid, insulin, sodium selenite, cholesterol, a collagen precursor, a trace element, 2-mercaptoethanol, and 3'-thioglycerol. These medium may further contain substances, as necessary, such as lipid, amino acid, protein, polysaccharide, vitamin, growth factor, low molecular weight compound, antibiotic, antioxidant, pyruvate, buffer, and inorganic salts. Example of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, MCDB201 medium, and combinations of these media. Examples of the serum includes, but not limited to, human serum, fetal bovine serum (FBS), bovine serum, bovine calf serum, goat serum, horse serum, porcine serum, sheep serum, rabbit serum, and rat serum. Serum, when used, may be added in 5 v/v% to 15 v/v%, preferably 10 v/v%, to the basal medium. Examples of the fatty acid include, but not limited to, linoleic acid, oleic acid, linolenic acid, arachidonic acid, myristic acid, palmitoyl acid, palmitic acid, and stearic acid. Examples of the lipid include, but not limited to, phosphatidyl serine, phosphatidylethanolamine, and phosphatidylcholine. Examples of the amino acid include, but not limited to, L-alanine, L-arginine, L-aspartic acid, L-asparagine, L-cysteine, L-cystine, L-glutamic acid, L-glutamine, and L-glycine. Examples of the protein include, but not limited to, ecotin, reduced glutathione, fibronectin, and β2-microglobulin. Examples of the polysaccharide include glycosaminoglycan, of which examples particularly include, but not limited to, hyaluronic acid and heparan sulfate. Examples of the growth factor include, but not limited to, platelet-derived growth factor (PDGF), basic fibroblast growth factor (bFGF), transforming growth factor-β (TGF-β), hepatocyte growth factor (HGF), epidermal growth factor (EGF), connective tissue growth factor (CTGF), and vascular endothelial growth factor (VEGF). Xeno-free medium, which does not contain xeno-derived components such as serum, is preferably used from the viewpoint of using the adipose-derived mesenchymal stem cells obtained in the present invention for cell transplantation. These media are available in the form of pre-prepared medium for mesenchymal stem cells (stromal cells) from, for example, PromoCell, Lonza, Biological Industries, Veritas Corporation, R&D Systems, Corning Inc., and Rohto Pharmaceutical Co., Ltd.

Subsequently, in the step (iii), cells in the cell suspension obtained in the step (ii) are cultured without being differentiated on a solid surface using the above-mentioned suitable cell medium under a suitable cell density and culture conditions. "Solid surface" in the present invention means any material which enables the binding of the adipose-derived mesenchymal stem cells of the present invention. In a specific embodiment, such a material is a plastic material treated to facilitate the binding of mammalian cells to the surface thereof. The shape of a culture vessel with a solid surface is not limited but a petri dish and a flask are preferably used. Cells are washed after incubation to remove non-binding cells and cell debris.

In the present invention, cells which finally stay in a binding state on a solid surface can be selected as a cell population of adipose-derived mesenchymal stem cells.

The selected cells may be analyzed on the surface antigens by a conventional method using flow cytometry or the like for confirming that they are adipose-derived mesenchymal stem cells of the present invention. Additionally, the cells may be tested on the differentiation potency into each cell line, and such a differentiation can be carried out by a conventional method.

Mesenchymal stem cells in the present invention can be prepared as described above but may be defined as the cells having the following characteristics;

    (1) adhesive property to plastic is demonstrated under culture conditions in standard medium,
    (2) surface antigens CD44, CD73, and CD90 are positive, and CD31 and CD45 are negative, and
    (3) differentiations into bone cells, adipocytes, and chondrocytes are feasible under culture conditions.

(Cryopreservation)

The mesenchymal stem cells in the present invention may be cells repeatedly cryopreserved and melted as necessary as long as having disease treatment effects. Cryopreservation in the present invention can be carried out by suspending mesenchymal stem cells in a cryopreservation solution well known by a person skilled in the art and cooling it. The suspension can be carried out by dissociating the cells using a dissociating agent such as trypsin, moving to a cryopreservation container, treating as necessary, and subsequently adding a cryopreservation solution thereto.

A cryopreservation solution may contain DMSO (Dimethyl sulfoxide) as a cryoprotective agent but DMSO has characteristics which induces differentiation of mesenchymal stem cells in addition to cytotoxicity and hence it is preferable to reduce a content of DMSO. Examples of the DMSO substitute include glycerol, propylene glycol, and polysaccharides. DMSO, when used, is contained in 5 v/v% to 20 v/v%, preferably 5 v/v% to 10 v/v%, and more preferably 10 v/v%. Additionally, additives described in WO2007/058308 may be contained. For such a cryopreservation solution, cryopreservation solutions provided by for example, BioVerde, NIPPON Genetics Co, Ltd, ReproCELL Inc., ZENOAQ, COSMO BIO, Kohjin Bio Co., Ltd., and Thermo Fisher Scientific may be used.

The above-mentioned suspended cells, when cryopreserved, may be stored at a temperature between -80°C to -100°C (for example, -80°C), and cryopreservation can be carried out using any freezer capable of achieving such a temperature. For avoiding abrupt temperature changes, a cooling rate may be controlled suitably using a programmed freezer but not particularly limited thereto. Cooling rate may suitably be selected depending on components of a cryopreservation solution and can be done according to a direction of a cryopreservation solution manufacturer.

Storage duration is not particularly limited in the upper limit as long as melted cells cryopreserved under the above conditions retain properties equal to before frozen, and examples include 1 week or more, 2 weeks or more, 3 weeks or more, 4 weeks or more, 2 months or more, 3 months or more, 4 months or more, 5 months or more, 6 months or more, 1 year or more, and more than these. Cell damages can be suppressed when stored at a lower temperature, and thus the cells may be moved to a gas phase on liquid nitrogen (-150°C or lower to -180°C or lower) and stored therein. Storage, when performed in a gas phase on liquid nitrogen, can be carried out using a preservation container well known by a person skilled in the art. For example, in the case of storing for 2 weeks or more, but not limited thereto, it is preferable to store in a gas phase on liquid nitrogen.

Melted mesenchymal stem cells may suitably be cultured until next cryopreservation. Culture of mesenchymal stem cells is carried out using the above-mentioned medium capable of culturing mesenchymal stem cells, and may be carried out at a culture temperature of 30 to 40°C, and preferably about 37°C in an atmosphere of air containing $CO_2$, but not limited thereto. A $CO_2$ concentration is 2 to 5%, and preferably about 5%. During culture, upon reaching a suitable confluency to a culture vessel (examples include cells occupying 50% to 80% of a culture vessel), the cells are dissociated using a dissociating agent such as trypsin and inoculated into a culture vessel separately provided in a suitable cell density to continue culture. When inoculating cells, typical examples of the cell density include 100 cells/cm$^2$ to 100,000 cells/cm$^2$, 500 cells/cm$^2$ to 50,000 cells/cm$^2$, 1,000 to 10,000 cells/cm$^2$, and 2,000 to 10,000 cells/cm$^2$. In a specific embodiment, a cell density is 2,000 to 10,000 cells/cm$^2$. It is preferable to adjust a period before a suitable confluency is reached in such a way as to be 3 days to 7 days. During culture, medium may suitably be replaced as necessary.

The cryopreserved cells can be melted using a method well known by a person skilled in the art. Examples include a method in which the cells are allowed to stand or shaken in a thermostatic chamber or a hot water bath at 37°C.

c) Mesenchymal stem cells of the present invention may be a cell in any condition such as a cell dissociated during culture and collected or a cell in a frozen condition in a cryopreservation solution. Same lots of cells obtained by expansion subdivided and cryopreserved are preferably used from the viewpoint of obtaining stable effects and good handleability. The cryopreserved mesenchymal stem cells may be melted immediately before use and, while being suspended in a cryopreservation solution, mixed directly with the above fibrinogen solution or thrombin solution, or may be mixed with the above fibrinogen solution or thrombin solution after suspended in an infusion or medium. Alternatively, the cells may be mixed directly with (a) the fibrinogen solution or (b) the thrombin solution after a cryopreservation solution is removed by a method such as centrifugation, or may be mixed with (a) the fibrinogen solution or (b) the thrombin solution after suspended in an infusion or medium. "Infusion" in the present invention herein refers to a solution used when treating human, and examples include, but not limited to, a physiological saline solution, a Japanese pharmacopoeia physiological saline, a 5% glucose solution, a Japanese pharmacopoeia glucose injection, a Ringer's solution, a Japanese pharmacopoeia Ringer's solution, a lactated Ringer's solution, an acetate Ringer's solution, No. 1 solution (starting solution), No. 2 solution (rehydration solution), No. 3 solution (maintenance solution), and No. 4 solution (post-operative recovery solution).

Mesenchymal stem cells may be suspended in either a) the fibrinogen solution or b) the thrombin solution. A cell concentration then is, from the viewpoint of treatment effects on a disease and easy preparation, $1 \times 10^5$ cells/mL to $5 \times 10^9$ cells/mL, preferably $5 \times 10^5$ cells/mL to $1 \times 10^9$ cells/mL, and more preferably $1 \times 10^6$ cells/mL to $5 \times 10^8$ cells/mL.

In the kit for preparing an agent for treating diseases of the present invention, a dose (dosage) of c) the mesenchymal stem cells can be different depending on the patient's conditions (body weight, age, symptoms, physical conditions and the like) and the agent for treating visceral diseases of the present invention and the like, but a larger dose tends to be preferable from the viewpoint of providing sufficient treatment effects on visceral diseases, whereas a smaller dose tends to be preferable from the viewpoint of suppressing the expression of side effects. Typically, for administration to an adult, a dose is $1 \times 10^3$ to $1 \times 10^{12}$ cells/dose, preferably $1 \times 10^4$ to $1 \times 10^{11}$ cells/dose, more preferably $1 \times 10^5$ to $1 \times 10^{10}$ cells/dose, further preferably $5 \times 10^5$ to $1 \times 10^9$ cells/dose, and particularly preferably $1 \times 10^6$ cells/dose to $5 \times 10^8$ cells/dose. Note that the above dose, as a single dose, may be administered several times or may be administered in several divided doses.

The kit for preparing an agent for treating diseases of the present invention may be administered with one or more other medicines. Examples of the other medicines include any medicines usable as a medicine for treating heart diseases such as ACE inhibitors, angiotensin II receptor antagonists, β-blockers, antiplatelet agents, Warfarin, calcium-channel blockers, nitric acid medicines, diuretics, HMG-CoA reductase inhibitors, and Ancaron.

The kit for preparing an agent for treating diseases of the present invention may contain pharmacologically acceptable carriers and additives according to a routine method depending on the purpose of usage and form thereof within the range in which the effects of the present invention are not affected. Examples of such carriers and additives include tonicity adjusting agents, thickeners, saccharides, sugar alcohols, antiseptics (preservatives), bactericides or antimicrobial agents, pH adjusters, stabilizers, chelating agents, oleaginous bases, gel bases, surfactants, suspending agents, binders, excipients, lubricants, disintegrants, foaming agents, fluidizing agents, dispersants, emulsifiers, buffers, solubilizing agents, antioxidants, sweeteners, acidulating agents, colorants, flavoring agents, perfumes, and cooling agents, but not limited thereto.

(Spraying apparatus)

The kit for preparing an agent for treating diseases of the present invention may include a spraying apparatus used when spraying, to a disease site, a) the fibrinogen solution or b) the thrombin solution containing mesenchymal stem cells and a) the fibrinogen solution or b) the thrombin solution not used in the suspension. The above spraying apparatus is not particularly limited but may be a commercial injection syringe equipped with an about 12 G to 24 G injection needle. Further, a spraying apparatus arranged in such a way that both solutions are mixed immediately before spraying by combining 2 syringes for the kit for preparing an agent for treating diseases of the present invention may be used.

(Method for creating the kit for preparing an agent for treating diseases)

A fibrinogen solution and a thrombin solution contained in separate containers respectively, mesenchymal stem cells suspension contained in a tube for freezing such as a cryotube, and a spraying apparatus are included together to be the kit for preparing an agent for treating diseases. The fibrinogen solution and the thrombin solution are preferably stored in a refrigerator and the mesenchymal stem cell suspension is preferably stored in a freezer. Note that other additives may be included therewith within the range in which the effects of the present invention are not affected. Additionally, an instruction manual can also be included in the present kit.

(Method for using the kit for preparing an agent for treating diseases)

Method for using the kit for preparing an agent for treating diseases of the present invention is not limited to a specific method but can be used, for example, as follow. The following preparation needs to be performed immediately before used for a surgery and the like.
Frozen mesenchymal stem cells are melted by heating at 4 to 50°C, preferably room temperature to 40°C, and more preferably room temperature to 37°C to obtain the mesenchymal stem cells suspended in a cryopreservation solution. A sufficient amount of the fibrinogen solution is added thereto and mixed. A sufficient amount of a high thrombin concentration solution is taken and added to a solvent containing calcium. For the solvent, an infusion, medium or a buffer is preferable. A ratio of fibrinogen to thrombin is, to 10 units of thrombin, 0.01 to 500 mg, preferably 0.1 to 50 mg, more preferably 0.3 to 30 mg, further preferably 0.5 to 10 mg, and particularly preferably 1 to 3.5 mg of fibrinogen. Each of the solutions is filled in a syringe for spraying and both solutions are sprayed substantially at the same time on a disease site to be gelled. "Sprayed substantially at the same time" used herein encompasses spraying, from respective syringes, respective solutions at any separate times, and mixing both solutions immediately before spraying and spraying on a disease site before the solution gels, in addition to spraying each solution on a disease site at the same time.
The gel obtained as described above needs to be formulated and prepared to demonstrate a suitable strength. A strength of the gel (composition) as the agent for treating diseases of the present invention is $10^2$ to $10^8$ dyn/cm$^2$, more preferably $10^3$ to $10^7$ dyn/cm$^2$, and further preferably $10^4$ to $10^6$ dyn/cm$^2$. Additionally, a viscosity of the gel (composition) is preferably 0 to 100 centipoise, more preferably 0 to 50 centipoise, and further preferably 25 to 40 centipoise. On the other hand, gelation time is preferably less than 60 seconds, more preferably less than 30 seconds, further preferably less than 15 seconds, and particularly preferably less than 5 seconds.
A pressure at the time of directly spraying on a disease site is 0.005 to 1.0 MPa, preferably 0.01 to 0.7 MPa, and more preferably 0.02 to 0.5 MPa. A spray rate at the time of directly spraying on a disease site is 0.01 to 10 mL/s, preferably 0.05 to 5.0 mL/s, and more preferably 0.5 to 1.0 mL/s. A spray shape at the time of directly spraying on a disease site may be any shape and not particularly limited, but from the viewpoint of easily coating a disease site, a circle is desirable, and from the viewpoint of safety and enabling the preparation of homogeneous gel, an area has a diameter of 7 cm, preferably a diameter of 5 cm, and more preferably a diameter of 3 cm. Note that humoral factors are secreted from the gel for 2 days or more, preferably 3 days or more, and more preferably 4 days or more. Examples of the humoral factor include HGF, VEGF, SDF-1, HIF-1, and CXCL-12. Further, cell survival in the gel of 3 days or more, preferably 5 days or more, and more preferably 7 days or more, can be confirmed.
The gel agent for treating diseases prepared using the kit for preparing an agent for treating diseases of the present invention contains mesenchymal stem cells and thus can suitably be used to treat various diseases. The agent is preferably used for, for example, visceral diseases, and specifically heart diseases, gastroduodenal diseases, small and large intestines diseases, liver diseases, biliary tract diseases, pancreatic diseases, renal diseases, lung diseases, mediastinal diseases, diaphragm diseases, pleural diseases, peritoneal diseases, and ocular diseases.
Examples of specific diseases include heart diseases such as myocardial infarction, heart failure, arrhythmia, palpitation, cardiomyopathy, ischemic cardiomyopathy, angina pectoris, congenital heart disease, valvular heart disease, myocarditis, familial hypertrophic cardiomyopathy, dilated cardiomyopathy, acute coronary syndrome, atherothrombosis, and restenosis; gastroduodenal diseases such as acute gastritis, chronic gastritis, gastroduodenal ulcer, stomach cancer, and duodenal cancer; small and large intestines diseases such as ischemic enterocolitis, ulcerative colitis, Crohn disease, simple ulcer, intestinal Behcet disease, small intestinal cancer, and large intestinal cancer; liver diseases such as fulminant hepatitis, chronic hepatitis, viral hepatitis, alcoholic hepatitis, liver fibrosis, cirrhosis, liver cancer, autoimmune hepatitis, fatty liver, medicine allergic hepatopathy, hemochromatosis, hemosiderosis, Wilson's disease, primary biliary cirrhosis, primary sclerosing cholangitis, biliary atresia, liver abscess, chronic active hepatitis, and chronic persistent hepatitis; biliary tract diseases such as acute cholecystitis, acute cholangitis, chronic cholecystitis, cholangiocarcinoma, and gallbladder carcinoma; pancreatic diseases such as acute pancreatitis, chronic pancreatitis, and pancreatic cancer; renal diseases such as acute nephritic syndromes, chronic nephritis, acute renal failure, and chronic renal failure; lung diseases such as pneumonia, emphysema, pulmonary fibrosis, interstitial pneumonia, pulmonary hypertension, pulmonary tuberculosis, pulmonary tuberculosis sequelae, acute respiratory distress syndrome, cystic fibrosis, chronic obstructive pulmonary disease, pneumoconiosis, aspiration pneumonia pulmonary fibrosis, acute upper respiratory infections, acute lower respiratory infection, pneumothorax, and diseases with injury to alveolar epithelium; mediastinal diseases such as mediastinal tumor, mediastinal cystic diseases, and mediastinitis; diaphragm diseases such as diaphragmatic hernia; pleural diseases such as pleurisy, empyema, pleura tumor, carcinomatous pleurisy, and pleural mesothelioma; peritoneal diseases such as peritonitis and peritoneal tumor; and ocular diseases such as Stevens-Johnson syndrome, ocular pemphigoid, thermochemistry injury, toxic epidermal necrolysis (TEN), pterygium, ortibal cellulitis, and retinal detachment. Of these, heart diseases on which sufficient treatment effects are confirmed to have been obtained are preferable, among which the agent can more suitably be used to treat myocardial infarction and heart failure.

Example of the subject to administration for using the kit for preparing an agent for treating diseases of the present invention include serosa, mucosa, conjunctival, and corneal epithelium, but serosa and mucosa are preferable, and serosa is more preferable. Examples of the administration route using the kit for preparing an agent for treating diseases of the present invention include direct administration to the organic surface, mucosal surface administration, serous membrane administration, intraorgan administration, oral administration, subcutaneous administration, intramuscular administration, intravenous administration, intra-arterial administration, intraspinal administration, sublingual administration, rectal administration, vaginal administration, ocular administration, nasal administration, inhalation, and dermal administration, but from the viewpoint of effectiveness of the agent for treating diseases of the present invention, direct administration to the organic surface, mucosal surface administration, serous membrane administration, and intraorgan administration are preferable, and from the viewpoint of lifting burdens of a subject, direct administration to the organic surface is more preferable.

Dosage of the gel agent for treating diseases prepared using the kit for preparing an agent for treating diseases is an amount at which treatment effects on a disease can be obtained when administered to a subject compared with a subject who is not administered. A specific dosage can suitably be determined depending on the subject's age, body weight, severity and symptoms of a disease, but, as an example, it is preferable to administer (spray) the agent in $1 \times 10^6$ to $1 \times 10^9$ cells per disease site in terms of the number of adipose-derived mesenchymal stem cells during a surgery, and this dosage may be administered (sprayed) at several sites or may be administered (sprayed) several times.

<Agent for treating disease>

An agent for treating diseases prepared using the above-mentioned kit for preparing an agent for treating diseases is also encompassed in the present invention. More specifically, the agent for treating diseases of the present invention is a gel agent for treating diseases prepared by suspending mesenchymal stem cells in a fibrinogen solution and directly spraying the obtained cell suspension and a thrombin solution, or suspending mesenchymal stem cells in a thrombin solution and directly spraying the obtained cell suspension and a fibrinogen solution, substantially at the same time on a disease site. For specific contents of the agent for treating diseases of the present invention, the description given in the section of the above-mentioned Kit for preparing an agent for treating diseases is applicable.

<Method for preparing the agent for treating diseases>

A method for preparing the agent for treating diseases using the above-mentioned kit for preparing an agent for treating diseases is also encompassed in the present invention. More specifically, the method for preparing the agent for treating diseases of the present invention is a method for preparing a gel agent for treating diseases, wherein mesenchymal stem cells are suspended in a fibrinogen solution and the obtained cell suspension and a thrombin solution are directly sprayed, or the mesenchymal stem cells are suspended in a thrombin solution and the obtained cell suspension and a fibrinogen solution are directly sprayed, substantially at the same time on a disease site. For specific contents of the method for preparing the agent for treating diseases of the present invention, the description given in the section of the above-mentioned Method for using the kit for preparing an agent for treating diseases is applicable.

<Method for treating diseases>

According to still another aspect of the present invention, provided is a method for treating diseases such as visceral diseases, wherein c) mesenchymal stem cells are suspended in a) a fibrinogen solution, and the obtained cell suspension and b) a thrombin solution are directly sprayed, or c) mesenchymal stem cells are suspended in b) a thrombin solution, and the obtained cell suspension and a) a fibrinogen solution are directly sprayed substantially at the same time on a disease site. According to the treatment method of the present invention, at a surgery of a visceral disease, when c) a mesenchymal stem cell is suspended in a) fibrinogen solution and the obtained cell suspension and b) a thrombin solution are directly sprayed, or c) mesenchymal stem cells are suspended in b) a thrombin solution, and the obtained cell suspension and a) a fibrinogen solution are directly sprayed, substantially at the same time on a disease site, function and the like at the disease site such as an organ can notably be improved. In the treatment method of the present invention, administration using a catheter is preferable from the viewpoint of quick post-operative recovery of a patient with a visceral disease and the like and reducing the size of an incision on a patient because a surgery can be performed only with a local anesthesia.

EXAMPLES

The present invention is specifically described with respect to the following Examples but is not limited to these Examples.

[1] Preparation of the agent for treating diseases of the present invention

Preparation of adipose-derived mesenchymal stem cells

After receiving consent from a human donor, subcutaneous adipose tissue obtained by a liposuction method is washed with a physiological saline solution. For achieving the destruction of extracellular matrix and isolation of the cells, collagenase (Roche) (solvent is a physiological saline solution) was added and shaken for 90 minutes at 37°C, and dispersed. Subsequently, the above suspension was centrifuged for 5 minutes at 800 g to obtain a precipitation of stromal vascular cell population. Serum-free medium for mesenchymal stem cells (Rohto Pharmaceutical Co., Ltd.) was added to the above cell precipitation, the cell suspension was centrifuged for 5 minutes at 400 g and resuspended, after removing the supernatant, in the serum-free medium for mesenchymal stem cells (Rohto Pharmaceutical Co., Ltd.), and the cells were inoculated in a flask. The cells were cultured at 37°C, 5% $CO_2$ for several days. Several days later, the culture was washed with PBS to remove remaining blood cells and adipose tissue contained in the culture medium and to obtain mesenchymal stem cells adhered to a plastic container.

Cryopreservation of adipose-derived mesenchymal stem cells

The obtained adipose-derived mesenchymal stem cells were dissociated using trypsin, moved to a centrifuge tube, and centrifuged for 5 minutes at 400 g to obtain a precipitation of cells. After removing the supernatant, a sufficient amount of a cell cryopreservation solution (STEM-CELLBANKER (ZENOAQ)) was added to suspend the cells. The cell-suspended solution was dispensed into a cryotube, subsequently stored at - 80°C in a freezer, and moved to a gas phase on liquid nitrogen to continue the storage.

Analysis on cell surface markers (flow cytometry)

Evaluations on various surface markers on the adipose-derived mesenchymal stem cell were carried out by flow cytometry. The adipose-derived mesenchymal stem cells were resuspended in FACS staining buffer. Antibodies used for the FACS analysis were FITC (fluorescein isothiocyanate) or PE (phycoerythrin)-labelled mouse anti-human antibodies CD11b, CD45, CD73, CD90, and a corresponding mouse IgG1 isotype control antibody. Cells were stained at room temperature for 30 minutes, subsequently washed, and analyzed using BD FACSCanto II (BD Biosciences, San Jose, CA). Data were analyzed using BD FACSDiva Software (BD Biosciences). As a result, the adipose-derived mesenchymal stem cell was negative for CD45 and positive for CD73 and CD90.

Preparation of a spray agent for treatment

A fibrinogen solution and a thrombin solution for spraying were prepared using Beriplast P Combi-Set Tissue adhesion (CSL Behring Co, Ltd.). The fibrinogen solution (Beriplast solution A) contains 80 mg/mL of fibrinogen, 60IU of factor XIII, and 5000KIE of bovine aprotinin. The thrombin solution (Beriplast solution B) contains 300 unit/mL of thrombin. Specifically, the adipose-derived mesenchymal stem cell was thawed immediately before spray suspended in HBSS (X1) in an amount shown in Table 1 below, and Beriplast solution A was added to prepare solution A. Similarly, Beriplast solution B was added to HBSS (X1) in an amount shown in Table 1 below to prepare solution B. These solutions were contained in separate syringes and prepared for the following transplantation test.

[Table 1]

| Solution A 2.0 mL | Beriplast solution A (Fibrinogen) | 400μL (32mg) |
|---|---|---|
| | ADSC in HBSS (X1) (adipose-derived mesenchymal stem cell) | $1×10^8$cells/1.6mL |
| Solution B 2.0 mL | Beriplast solution B (Thrombin) | 400μL (120 units) |
| | HBSS (X1) | 1.6mL |

[2] Transplantation test

Protocol of transplantation test

An ameroid constrictor was placed on the left anterior descending coronary artery of 12 female mini pigs (body weight 20 to 25 kg) to cause myocardial infarction. The mini pigs were randomly divided into 2 groups, adipose-derived mesenchymal stem cells transplantation group (ADSC group) and a sham surgery group (control) (each n = 6). Four weeks after ameroid constrictor placement, either administration of the above spray agent for treatment (transplantation of adipose-derived mesenchymal stem cells) or the sham surgery was carried out.

For the mini pigs of the ADSC group, the solutions A and B having the compositions shown in the above Table 1 and sprayed at the same time post- median sternotomy covering/coating the surface of a site at which myocardial infarction has been developed (heart surface) to coat the disease site. The sprayed mixed solution gelled immediately after spray. During the sham surgery, only median sternotomy and opening the pericoordic sac was carried out and the administration of spray agent for treatment (transplantation of adipose-derived mesenchymal stem cells) was not carried out.

Echocardiography, MRI, NH3-PET, and heart catheterization were carried out before transplantation (baseline) and 4 weeks and 8 weeks after transplantation. At the final stage of this study, the animals were humanely sacrificed at the 8 weeks after cells transplantation for the sake of histological and biochemical analysis of the cardiac tissue. All the animals were not immunosuppressed. Hereinafter, the transplantation test is described in detail.

Creation of myocardial infarction model pig and adipose-derived mesenchymal stem cells transplantation experiment

Twelve female mini pigs (body weight 20 to 25 kg) were preanesthetized with ketamine hydrochloride (20 mg/kg; DAIICHI SANKYO, Tokyo, Japan) and xylazine (2 mg/kg; Bayer HealthCare, Leverkusen, Germany), intubated endotracheally to maintain general anesthesia by continuous reflux of propofol (6 mg/kg/h; Astra-Zeneca K.K., Osaka Japan) and vecuronium bromide (0.05 mg/kg/h; DAIICHI SANKYO). Pericardial cavity was exposed by left thoracotomy via the 4th intercostal space. An ameroid constrictor (COR-2.50-SS; Research Instruments SW, Escondido, CA) was positioned around the left anterior descending (LAD) coronary artery close to the bifurcation of the left circumflex coronary artery, and layered closure of muscles and the skin were performed. Subsequently, the mini pigs were recovered in a temperature-controlled cage.

Four weeks after ameroid constrictor placement, median sternotomy was performed under general anesthesia and either adipose-derived mesenchymal stem cells transplantation or a sham surgery was carried out. The site at which myocardial infarction has been developed can be visually confirmed based on surface scars and abnormal wall motions. Specifically, in the ADSC group, the solutions A and B having the compositions shown in the above Table 1 (containing $1 × 10^8$ of adipose-derived mesenchymal stem cells) and contained in separate syringes respectively were directly sprayed at the same time on the surface of a site at which myocardial infarction has been developed (heart surface) to coat the disease site. The sprayed mixed solution gelled immediately after sprayed. The mini pigs were recovered in an individual temperature-controlled cage.

Effect of adipose-derived mesenchymal stem cells transplantation

Effects of the above spray agent for treatment (transplantation of adipose-derived mesenchymal stem cells) on the myocardial infarction model pigs were evaluated by echocardiography, MRI, NH3-PET, and heart catheterization. Each of the evaluation methods is described below.

(Echocardiography)

Mini pigs were anesthetized as described above. Echocardiography was carried out using a commercial echo apparatus (HITACHI: PROSOUND F75 Premier CV). An 8.0-MHz annular array transducer was used to evaluate the heart. Mini pigs were tested in the left lateral decubitus position. LV end-diastolic and end-systolic diameters (LVDd and LVDs respectively) were measured. LV end-diastolic and end-systolic volumes (LVEDV and LVESV) were calculated by Teichholz's formula. LV ejection fraction (LVeF) was calculated from the following formula. The results are shown in Table 2.

$$LVEF\ (\%) = 100 \times (LVEDV\text{-}LVESV) / (LVEDV)$$

As shown in Table 2, the mini pigs to which the above spray agent for treatment (transplantation of adipose-derived mesenchymal stem cells) was applied demonstrated improvement in cardiac output.

($^{13}$N-NH$_3$-PET)

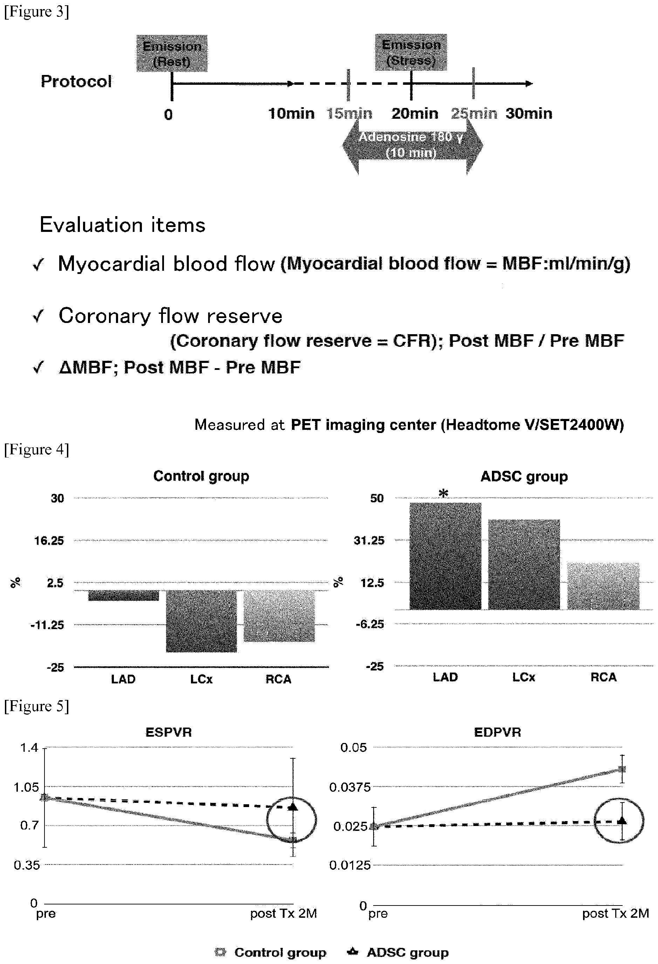

Evaluation on myocardial blood flow was carried out by $^{13}$N-NH$_3$-PET according to the protocol shown in Fig. 3. $^{13}$N-NH$_3$-PET data were acquired according to a 1-D single-session stress-rest protocol. More specifically, adenosine stimulation to peripheral venous and intravenous administration of 700 to 900 MBq of $^{13}$N-NH$_3$ were carried out and transmission scan was carried out for the attenuation correction of an advance PET scanner (Headtome V/SET2400W; manufactured by Shimadzu Corporation). Adenosine was injected at 180 $\mu$g/kg/min over a period of 10 minutes and scanning was started in the middle of this stimulation.

Data analysis

Calculation of myocardial blood flow (MBF) value from the $^{13}$N-NH$_3$-PET image was carried out using a commercial PMOD software package (version 3.4, PMOD Technologies LLC, Zurich, Switzerland). As in the vertical and horizontal long axis directions, the image was also resliced along the short axis and reconstructed. Necessary myocardial regions of right ventricular cavity, left ventricular cavity, and left ventricle were automatically analyzed and minimal modification was made as necessary by a technician experienced in the heart anatomy to avoid contaminants from outside. Local $^{13}$N-NH$_3$ uptake was evaluated using the American Heart Association 17-segment model. Time-activation curve of myocardium and blood pool produced from a dynamic frame was matched to a tracer kinetics model. The DeGrado 1-compartment model, which assumes that there is no metabolic trapping, was employed. Rest MBF and stress MBF were expressed in each segment and territory, and myocardial flow reserve (MFR) was calculated in terms of a ratio of the rest MBF to stress MBF. It is considered that MFR of 2.5 or more is normal, 2.0 to 2.5 is gray zone, and less than 2.0 is decreased. Pretreatment and post-treatment MFR were compared.
As shown in Fig. 4, the mini pigs to which the above spray agent for treatment (transplantation of adipose-derived mesenchymal stem cells) was applied demonstrated about 50% improvement in coronary flow reserve in the LAD legion.

(Heart catheterization)

Mini pigs were anesthetized and ventilated before treatment and 8 weeks after treatment. A tourniquet was placed around the inferior vena cava and an LV preload was controled. A conductance and pressure-tip catheter (PV combination catheter: CA-41063-PN, 4Fr) was inserted into the left ventricle via the right ventricular artery at pretreatment and via the LV apex toward the aortic valve at 8 weeks after transplantation. A conductance system and a pressure transducer controller (Conduct NT Sigma 5DF plus analysis system: CFL-M) were set. The conductance, pressure and intracardial electrocardiographs signals were analyzed using Inca software (CD Leycom, Neth). The baseline was initially measured under stable hemodynamic conditions, and subsequently the pressure volume loop was drawn during inferior vena caval occlusion and analyzed. The following indexes: dP/dtmax, dP/dtmin, the time constant of isovolumic relaxation ($\tau$), end-diastolic pressure-volume relationship (EDPVR), and end-systolic pressure-volume relationship (ESPVR) were calculated. The results are shown in Table 5.

* PV Combination catheter (CA-41063-PN, 4Fr)

* Conduct NT Sigma 5DF plus analysis system (CFL-M)
* CD Leycom Neth.

As shown in Fig. 5, the mini pigs to which the above spray agent for treatment (transplantation of adipose-derived mesenchymal stem cells) was applied had the contractility and diastolic function maintained when compared with the control group.

(magnetic resonance imaging: MRI)

Pigs were given intramuscular injection of ketamine 10 mg/kg + xylazine 2 mg/kg and atropine 1A and sedated. After sedation, the pigs were shaved and a incubated tube was inserted. A peripheral venous line was inserted before a pig was laid on an examining table in a magnetic resonance imaging (MRI) room. In the supine position, distribution pipes of anesthesia equipment were connected to the tracheal tube, and electrocardiogram electrical codes were adhered to the chest. Isoflurane was used as an anesthesia and a concentration was maintained at 1 to 2%. A coil was fitted over the chest region and the pig was moved to a tunnel of the MRI apparatus (Sigma EXCITE XI Twin Speed 1.5T Ver. 11.1, GE Healthcare).

Two types of imaging, Cine MRI showing heart movement and delayed gadolinium-enhanced MRI, were carried out. In Cine MRI, images of the heart region are obtained in segments along the axial plane using two-dimensional Fiesa imaging. From the data collected above, 20 segments per heart beat are obtained along the base from the apex of the LV using 2D Fiesa imaging. The diastolic phase was selected from the collected data and images using 2D Fiesa imaging were obtained using the major axis segments covering the mitral valve region from the apex. From the data of longitudinal segments, segments taken during diastole were selected and segments vertical to the long axis of the left ventricular were used. Ten to twelve segments (short-axis segments) are obtained using 2D Fiesa imaging from the apex of heart at intervals of 6 to 8 mm. In the delayed MRI, images using Fast GRE were obtained in 5 to 15 minutes from administration of a contrast agent (Omniscan intravenous injection 32%, 0.25 mL/kg). Infarct regions and fibrosis-formed regions were depicted by a high signal intensity; these regions must visually be different from normal regions. In each pig, baseline and follow-up 4-chamber views, a 2-chamber view, a short-axis view, and a long-axis view of the papillary muscle were retrieved from the database for further analysis. After manual tracing of the endocardial contour on an end-diastolic frame, a dedicated software (TOMTEC Inc.) automatically tracked the endo- and epicardial contours on the other frames of the cine loop. Suitable tracking was verified in real-time and corrected by adjusting the region of interest or manually correcting the contour. With these adjustments, the 2D-strain software finally tracked the 16 segments of the LV myocardium in all the pigs. Peak systolic longitudinal, radial, and circumferential strain were measured at end-systole in all views and averaged.

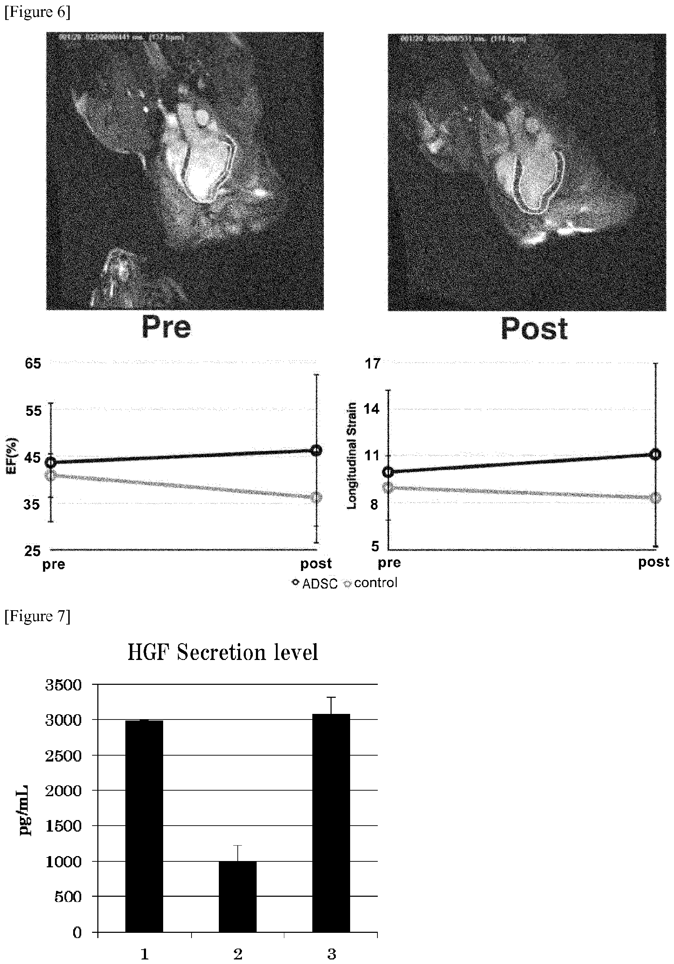

As shown in Fig. 6, in the mini pigs to which the above spray agent for treatment (transplantation of adipose-derived mesenchymal stem cells) was applied both the EF (%) value and the longitudinal strain were maintained when compared with the control group.

[3] Secretory factor from adipose-derived mesenchymal stem cells -1

Adipose-derived mesenchymal stem cells were grafted (gellated) with fibrinogen and thrombin as in in vivo experiment. Specifically, a solution A preparation containing 14 $\mu$L of solution A (fibrinogen) of Beriplast (registered trademark)P Combi-Set tissue adhesion 3mL formulation (CSL Behring Co, Ltd.), 6 $\mu$L of HBSS (gibco, Cat. No: 14174-103, Lot: 1776567), and $1 \times 10^6$ cells of adipose-derived mesenchymal stem cells were added dropwise onto each well of hydrophobic 6-well plates (Thermo Scientific, 140675), and a solution B preparation containing 14 $\mu$L of solution B (thrombin) of Beriplast (registered trademark) P Combi-Set tissue adhesion 3 mL formulation (CSL Behring Co, Ltd.) and 6 $\mu$L of HBSS (gibco, Cat. No: 14174-103, Lot: 1776567) was added with stirring to the solution A preparation in the form of water droplets to prepare a gel. The gel was placed in 6-Well Clear Multiple Well Plates (Corning (registered trademark) CellBIND (registered trademark), 3335), 5 mL of medium (MSCGM BulletKit (MSCGM Mesenchymal Stem Cell Growth Medium BulletKit), manufactured by Lonza, Cat. No: PT-3001) was added, cultured in a $CO_2$ incubator (37°C, 5%$CO_2$), and the supernatant thereof was collected after 72 hours. Cytokine in the collected culture supernatant was measured by ELISA (ELISA kit (Quantikine ELISA Human HGF (R&D System, Cat. No: DHG00), Quantikine ELISA Human VEGF (R&D System, Cat. No: DHE00)). The adipose-derived mesenchymal stem cell was confirmed to have secreted VEGF and HGF in the gel.

[4] Secretory factor from adipose-derived mesenchymal stem cells -2

As in above, a solution A preparation containing 140 $\mu$L of solution A (fibrinogen) of Beriplast (registered trademark)P Combi-Set tissue adhesion 3 mL formulation (CSL Behring Co, Ltd.), 60 $\mu$L of HBSS (gibco, Cat. No:

14174-103, Lot: 1776567), and $5 \times 10^6$ cells of adipose-derived mesenchymal stem cells and a solution B preparation containing 140 μL solution B (thrombin) of Beriplast (registered trademark)P Combi-Set tissue adhesion 3 mL formulation (CSL Behring Co, Ltd.) and 60 μL of HBSS (gibco, Cat. No: 14174-103, Lot:1776567) were used to prepare a gel, subsequently the gel was cultured in 5 mL of medium, and the supernatant thereof was collected after 6 hours, 24 hours, 48 hours, and 72 hours. Cytokines in the collected culture supernatant were measured by ELISA (ELISA kit (Quantikine ELISA Human HGF (R&D System, Cat. No: DHG00), Quantikine ELISA Human VEGF (R&D System, Cat. No: DHE00)). The adipose-derived mesenchymal stem cells were confirmed to have continuously secreted VEGF and HGF in the gel after 6 hours to 72 hours.

[5] Secretory factor from adipose-derived mesenchymal stem cells -3

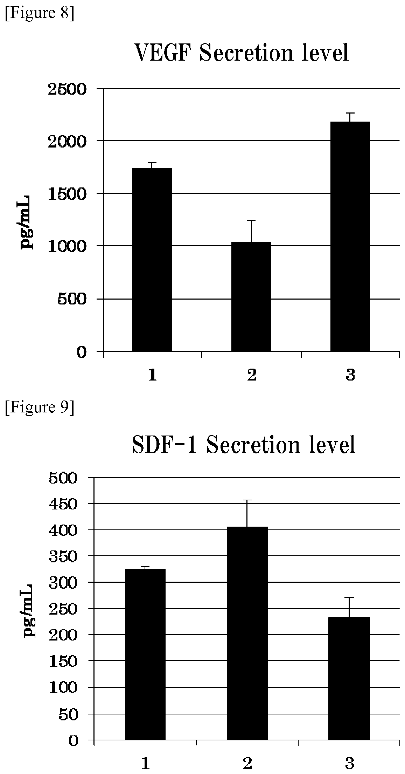

A solution A preparation containing 6 μL of solution A (fibrinogen, hereinafter referred to as "solution A") of Beriplast (registered trademark) P Combi-Set tissue adhesion 3 mL formulation (CSL Behring Co, Ltd.), 14 μL of HBSS (gibco, Cat. No: 14174-103, Lot: 1776567, hereinafter referred to as "HBSS"), and $1 \times 10^6$ cells of adipose-derived mesenchymal stem cells and a solution B preparation containing 6 μL of solution B (thrombin, hereinafter referred to as "solution B") of Beriplast (registered trademark)P Combi-Set tissue adhesion 3 mL formulation (CSL Behring Co, Ltd.) and 14 μL of HBSS were used to prepare a gel (gel sample 1). Similarly, a solution A1 preparation containing 20 μL of solution A and $1 \times 10^6$ cells of adipose-derived mesenchymal stem cells and a B1 solution preparation containing 2 μL of solution B and 18 μL of HBSS were used to prepare gel sample 2, and a A2 solution preparation containing 4.5 μL of solution A, 15.5 μL of HBSS, and $1 \times 10^6$ cells of adipose-derived mesenchymal stem cells and a B2 solution preparation containing 6.5 μL of solution B and 11.5 μL of HBSS were used to prepare gel sample 3. Gel samples 1 to 3 were cultured in 5 mL of medium, the supernatant thereof was collected after 72 hours, and cytokine in the collected culture supernatant was measured by ELISA (ELISA kit (Quantikine ELISA Human HGF (R&D System, Cat. No: DHG00), Quantikine ELISA Human VEGF (R&D System, Cat. No: DHE00)), Human CXCL12/SDF-1 alpha Quantikine ELISA Kit (R&D System, Cat. No: DSA00). The adipose-derived mesenchymal stem cells were confirmed to have secreted VEGF, HGF, and SDF-1 in the gel (Figs. 7 to 9).

[6] mRNA Expression level of adipose-derived mesenchymal stem cells

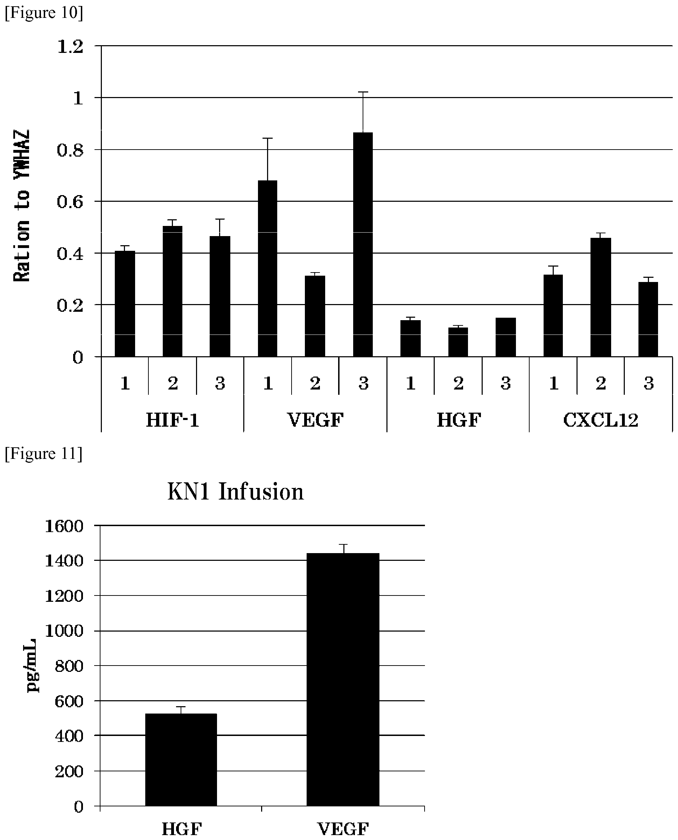

As in [5], a solution A preparation containing 6 μL of solution A (fibrinogen, hereinafter referred to as "solution A") of Beriplast (registered trademark)P Combi-Set tissue adhesion 3 mL formulation (CSL Behring Co, Ltd.), 14 μL of HBSS (gibco, Cat. No: 14174-103, Lot: 1776567, hereinafter referred to as "HBSS"), and $1 \times 10^6$ cells of adipose-derived mesenchymal stem cells and a solution B preparation containing 6 μL of solution B (thrombin, hereinafter referred to as "solution B") of Beriplast (registered trademark)P Combi-Set tissue adhesion 3 mL formulation (CSL Behring Co, Ltd.) and 14 μL of HBSS were used to prepare a gel (gel sample 1). Similarly, a A1 solution preparation containing 20 μL of solution A and $1 \times 10^6$ cells of adipose-derived mesenchymal stem cells and a B1 solution preparation containing 2 μL of solution B and 18 μL of HBSS were used to prepare gel sample 2, and a A2 solution preparation containing 4.5 μL of solution A, 15.5 μL of HBSS, and $1 \times 10^6$ cells adipose-derived mesenchymal stem cells and a B2 solution preparation containing 6.5 μL of solution B and 11.5 μL of HBSS were used to prepare gel sample 3. Gel samples 1 to 3 were cultured in 1 mL of medium, and mRNA was collected after 24 hours using RNeasy Fibrous Tissue Mini Kit (QIAGEN, Cat. No: 74704). cDNA was created from the collected mRNA using PrimeScript (trademark)RT Master Mix (TaKaRa, Cat. No: RR036A). A mixed solution of the created cDNA, Probe qPCR Mix (TaKaRa, Cat. No: RR391A), and Primer/Probe from TaqMan (registered trademark) Gene Expression Assays (Applied biosystems) shown in a list was dispensed in a 96-well plate, and qPCR was carried out with a Fast protocol of ViiA (trademark) 7 real-time PCR system (Applied biosystems), whereby the expressions of mRNA shown in Fig. 10 were confirmed. Note that the analysis was carried out by Comparative CT method with YWHAZ as an endogenous control. The primers used are shown in Table 2 below.

[Table 2]

| Name of genes | Assay ID |
|---|---|
| hypoxia inducible factor 1, alpha subunit (basic helix-loop-helix transcription factor) | Hs00153153_m1 |
| vascular endothelial growth factor A | Hs00900055_m1 |
| hepatocyte growth factor (hepapoietin A; scatter factor) | Hs00300159_m1 |
| chemokine (C-X-C motif) ligand 12 | Hs03676656_mH |
| tyrosine 3-monooxygenase/tryptophan 5-monooxygenase activation protein, zeta polypeptide | Hs03044281_g1 |

[7] Secretory factor from adipose-derived mesenchymal stem cells -4

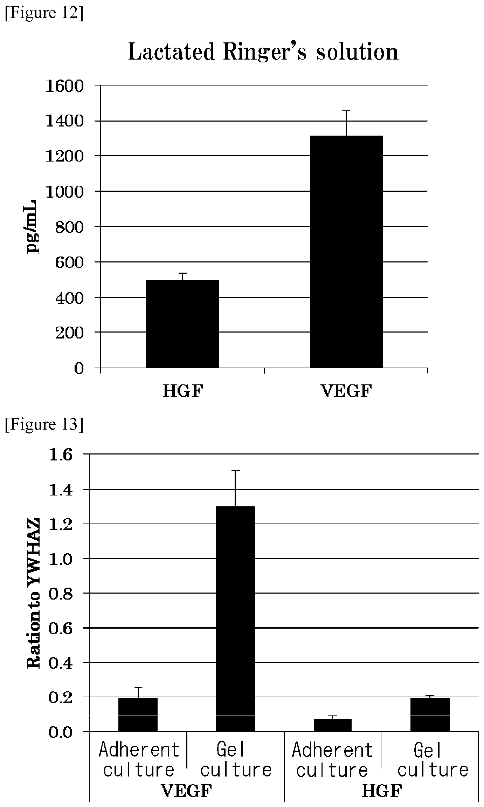

As in [5], a solution A preparation containing 6 μL of Beriplast solution A (fibrinogen, hereinafter referred to as "solution A"), 14 μL of HBSS (gibco, Cat. No: 14174-103, Lot: 1776567, hereinafter referred to as "HBSS"), and 1 × $10^6$ cells of an adipose-derived mesenchymal stem cells and a solution B preparation containing 6 μL of solution B (thrombin, hereinafter referred to as "solution B") of Beriplast (registered trademark)P Combi-Set tissue adhesion 3 mL formulation (CSL Behring Co, Ltd.) and 14 μL of HBSS were used to prepare a gel (gel sample 1). Similarly, a solution A1 preparation containing 6 μL of solution A, 14 μL of KN1 infusion (Otsuka Pharmaceutical Factory, Inc., Cat. No: 1964, Lot: K6F77, hereinafter referred to as "KN1 infusion"), and 1 × $10^6$ cells of adipose-derived mesenchymal stem cells and a solution B1 preparation containing 6 μL of solution B and 14 μL of KN1 infusion were used to prepare gel sample 2, and a solution A2 preparation containing 6 μL of solution A, 14 μL of lactated Ringer's solution (I'rom Co., Ltd., Cat. No: A1A82, Lot: 1AA3A, hereinafter referred to as "lactated Ringer's solution"), and 1 × $10^6$ cells of adipose-derived mesenchymal stem cells and a solution B2 preparation containing 6 μL of solution B and 14 μL of lactated Ringer's solution were used to prepare gel sample 3. Gel samples 1 to 3 were cultured in 5 mL of medium, the supernatant thereof was collected after 72 hours, and cytokine in the collected culture supernatant was measured by ELISA (ELISA kit (Quantikine ELISA Human HGF (R&D System, Cat. No: DHG00), Quantikine ELISA Human VEGF (R&D System, Cat. No: DHE00)). The adipose-derived mesenchymal stem cells were confirmed to have secreted VEGF and HGF even in the gel where KN1 infusion and lactated Ringer's solution were used (Figs. 11 and 12).

[8] mRNA Expression level of adipose-derived mesenchymal stem cells

As in [5], a solution A preparation containing 6 μL of solution A (fibrinogen, hereinafter referred to as "solution A") of Beriplast (registered trademark)P Combi-Set tissue adhesion 3 mL formulation (CSL Behring Co, Ltd.) and 14 μL of HBSS (gibco, Cat. No: 14174-103, Lot: 1776567, hereinafter referred to as "HBSS" and a solution B preparation containing 6 μL of solution B (thrombin, hereinafter referred to as "solution B") of Beriplast (registered trademark)P Combi-Set tissue adhesion 3 mL formulation (CSL Behring Co, Ltd.), 14 μL of HBSS, and 1 × $10^6$ cells of adipose-derived mesenchymal stem cells were used to prepare a gel. The prepared gel was cultured in 1 mL of medium (Lonza, Cat. No: PT-3001), and mRNA was collected after 24 hours using RNeasy Fibrous Tissue Mini Kit (QIAGEN, Cat. No: 74704). As adherent culture cells, 4.75 × $10^4$ cells of adipose-derived mesenchymal stem cells were cultured in 1 mL of medium (Lonza, Cat. No: PT-3001) using a 24-well plate (Corning, Cat. No: 3337), and mRNA was collected after 24 hours using RNeasy Fibrous Tissue Mini Kit (QIAGEN, Cat. No: 74106). cDNA was created from the collected 2 types of mRNA using PrimeScript (trademark)RT Master Mix (TaKaRa, Cat. No: RR036A). The created cDNA, Probe qPCR Mix (TaKaRa, Cat. No: RR391A), and Primer/Probe (TaqMan (registered trademark) Gene Expression Assays (Applied biosystems)) shown in Table 3 were mixed and dispensed in a 96-well plate, and qPCR was carried out with a Fast protocol of ViiA (tradename) 7 real-time PCR system (Applied biosystems). As shown in Fig. 13, the cells in the gel were confirmed to have higher expression levels of mRNA of VEGF and HGF when compared with the adherent culture cells. Note that the analysis was carried out by Comparative CT method with YWHAZ as an endogenous control.

[Table 3]

| Name of genes | Assay ID |
|---|---|
| vascular endothelial growth factor A | Hs00900055_m1 |
| hepatocyte growth factor (hepapoietin A; scatter factor) | Hs00300159_m1 |
| tyrosine 3-monooxygenase/tryptophan 5-monooxygenase activation protein, zeta polypeptide | Hs03044281_g1 |

INDUSTRIAL APPLICABILITY

According to the kit for preparing an agent for treating diseases of the present invention, at a surgery of a disease such as heart failure, when mesenchymal stem cells are suspended in a fibrinogen solution or a thrombin solution and the obtained cell suspension and the fibrinogen solution or the thrombin solution not used in the suspension are directly sprayed on a disease site substantially at the same time, functions and the like at the disease site such as heart can be notably improved. The mesenchymal stem cells hardly cause a rejection even to an allogeneic subject and thus donor's cells with confirmed treatment effects in advance is expanded, cryopreserved and can be used as the mesenchymal stem cells for the kit for preparing an agent for treatment of the present invention. For this reason, when compared with the case where autologous mesenchymal stem cells are prepared and used, commercialization is easier and consistent effects can be easily achieved, hence advantageous.

**Claims**

1.  A kit for preparing an agent for treating diseases comprising:
    a) a fibrinogen solution, b) a thrombin solution, and c) mesenchymal stem cells in separate forms.

2.  The kit for preparing an agent for treating diseases according to claim 1, wherein c) the mesenchymal stem cells are allogeneic to a subject.

3.  The kit for preparing an agent for treating diseases according to claim 1 or 2, wherein, when in use, c) the mesenchymal stem cells are suspended in either a) the fibrinogen solution or b) the thrombin solution, and then the obtained cell suspension is directly sprayed on a disease site substantially at the same time with either b) the thrombin solution or a) the fibrinogen solution that is not used in the suspending step.

4.  The kit for preparing an agent for treating diseases according to claim 3, wherein the directly sprayed solution gels on the disease site to be an agent for treating diseases.

5.  The kit for preparing an agent for treating diseases according to any one of claims 1 to 4, wherein c) the mesenchymal stem cells are frozen cells.

6.  The kit for preparing an agent for treating diseases according to any one of claims 1 to 5, wherein the disease is a visceral disease or an ocular disease.

7.  The kit for preparing an agent for treating diseases according to any one of claims 1 to 6, wherein c) the mesenchymal stem cells are prepared to be contained in $1 \times 10^6$ to $1 \times 10^9$ cells/mL in the gel.

8.  A gel agent for treating diseases prepared by suspending c) mesenchymal stem cells in either a) a fibrinogen solution or b) a thrombin solution and directly spraying the obtained cell suspension on a disease site substantially at the same time with either a) the fibrinogen solution or b) the thrombin solution that is not used in the suspending step.

9.  A method for preparing a gel agent for treating diseases, wherein c) mesenchymal stem cells suspended in either a) a fibrinogen solution or b) a thrombin solution and then the obtained cell suspension is directly sprayed on a disease site substantially at the same time with either a) the fibrinogen solution or b) the thrombin solution that is not used in the suspending step.

[Figure 1]

[Figure 2]

[Figure 3]

**Protocol**

Emission (Rest)

Emission (Stress)

0              10min  15min  20min  25min  30min

Adenosine 180 γ (10 min)

## Evaluation items

✓ Myocardial blood flow (Myocardial blood flow = MBF:ml/min/g)

✓ Coronary flow reserve

(Coronary flow reserve = CFR); Post MBF / Pre MBF

✓ ΔMBF; Post MBF - Pre MBF

Measured at **PET imaging center (Headtome V/SET2400W)**

[Figure 4]

[Figure 5]

◻ Control group   ▲ ADSC group

[Figure 6]

Pre

Post

◇ ADSC ○ control

[Figure 7]

HGF Secretion level

[Figure 8]

## VEGF Secretion level

[Figure 9]

## SDF-1 Secretion level

[Figure 10]

[Figure 11]

KN1 Infusion

[Figure 12]

Lactated Ringer's solution

[Figure 13]

## INTERNATIONAL SEARCH REPORT

| | International application No. |
|---|---|
| | PCT/JP2017/037263 |

**A. CLASSIFICATION OF SUBJECT MATTER**
Int. Cl. A61K35/28(2015.01)i, A61K9/10(2006.01)i, A61K9/12(2006.01)i,
A61K38/00(2006.01)i, A61K38/48(2006.01)i, A61P9/00(2006.01)i,
A61P27/02(2006.01)i, A61P43/00(2006.01)i
According to International Patent Classification (IPC) or to both national classification and IPC

**B. FIELDS SEARCHED**

Minimum documentation searched (classification system followed by classification symbols)
Int. Cl. A61K35, A61K9, A61K38, A61P9, A61P27, A61P43

Documentation searched other than minimum documentation to the extent that such documents are included in the fields searched
Published examined utility model applications of Japan 1922-1996
Published unexamined utility model applications of Japan 1971-2017
Registered utility model specifications of Japan 1996-2017
Published registered utility model applications of Japan 1994-2017

Electronic data base consulted during the international search (name of data base and, where practicable, search terms used)
Int. Cl. JSTPlus/JMEDPlus/JST7580 (JDreamII), CAplus/MEDLINE/EMBASE/BIOSIS
(STN)

**C. DOCUMENTS CONSIDERED TO BE RELEVANT**

| Category* | Citation of document, with indication, where appropriate, of the relevant passages | Relevant to claim No. |
|---|---|---|
| X | JP 2010-510212 A (ABBOTT CARDIOVASCULAR SYSTEMS INC.) 02 April 2010, claims, paragraphs [0001], [0019]-[0025] | 1, 6-7 |
| Y | & JP 5313154 B2 & JP 2010-516349 A & JP 5542449 B2 & US 2008/0119385 A1, claims, paragraphs [0001], | 2, 5 |
| A | [0033]-[0040] & US 2009/0022817 A1 & US 2014/0348800 A1 & US 2015/0209473 A1 & WO 2008/063418 A2 & WO 2008/089452 A2 & EP 2101831 A2 & EP 2109469 A2 & EP 2452699 A1 | 3-4, 8-9 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| | |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2017/037263 |

C (Continuation). DOCUMENTS CONSIDERED TO BE RELEVANT

| Category* | Citation of document, with indication, where appropriate, of the relevant passages | Relevant to claim No. |
| --- | --- | --- |
| X | JP 2004-161649 A (ASAHI MEDICAL CO.) 10 June 2004, claims, paragraphs [0026], [0030] (Family: none) | 1, 3-4, 6-9 |
| Y | | 2, 5 |
| Y | JP 2011-529957 A (CELLERIX SA) 15 December 2011, paragraph [0064] & JP 2016-40256 A & US 2012/0027730 A1, paragraph [0077] & WO 2010/015929 A2 & EP 2328594 A2 & EP 3095449 A1 | 2 |
| Y | WO 2013/146992 A1 (JCR PHARMACEUTICALS CO., LTD.) 03 October 2013, paragraphs [0003], [0005] & US 2015/0050731 A1, paragraphs [0003], [0005] & EP 2832850 A1 | 2, 5<br><br>5 |
| Y | JP 2009-242265 A (JCR CO., LTD.) 22 October 2009, paragraph [0009] & US 2009/0246181 A1, paragraph [0009] & US 2011/0070207 A1 & EP 2105498 A1 | |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003306434 A
- JP S58185162 A
- JP S57153645 A

- JP 2004161649 A
- US 6777231 B
- WO 2007058308 A